# EUROPEAN PATENT APPLICATION

(11) **EP 2 708 356 A1**
(43) Date of publication of application: **19.03.2014**
(21) Application number: 12781662.7
(22) Date of filing: 01.05.2012
(51) Int. Cl.: B32B 27/00, B05D 1/36, A61L 24/00

(54) **THIN FILM WITH BASE AND METHOD FOR PRODUCING SAME**

(30) Priority: 12.05.2011 JP 2011107327; 12.05.2011 JP 2011107330
(71) Applicant: Hitachi Chemical Company, Ltd., Chiyoda-ku Tokyo 100-6606 (JP)
(72) Inventor: KAMO, Kazuyuki, Tsukuba-shi, Ibaraki 300-4247 (JP); TAKANE, Nobuaki, Tsukuba-shi, Ibaraki 300-4247 (JP)
(74) Representative: HOFFMANN EITLE
(86) International application number: PCT/JP2012/061576
(87) International publication number: WO 2012/153682

(57) **Abstract**

The present invention provides a thin film with a base, comprising a base and a thin film formed on the base, wherein the thin film comprises a layer A formed by using a solution containing a polycation; and a layer B formed by using a solution containing a polyanion and having a pH of 1.6 to 5.4.

## Description

### Technical Field

The present invention relates to a thin film with a base and a method for producing the same.

### Background Art

As a tissue adhesive used in surgery, so-called fibrin glue comprising fibrinogen, blood coagulation factor XIII, and thrombin has been known, for example. However, the fibrin glue has problems in that pathogens such as a virus may be contained therein because fibrinogen from human blood plasma is used and the handleability is poor because there is a need to mix several materials just before use.

Also, as a tissue adhesive used in surgery, a cyanoacrylate tissue adhesive is commercially available. The cyanoacrylate tissue adhesive has a high curing speed and high adhesive strength, however, there are pointed out problems that the cyanoacrylate tissue adhesive has poor flexibility after cured and may produce toxic formaldehyde when degraded in vivo.

Meanwhile, it has been widely known that, when a cationic polymer and an anionic polymer are mixed under the presence of water, a polyion complex is formed rapidly, and the polyion complex is used in a wide variety of fields including medicinal products and medical devices.

For example, Patent Literature 1 discloses an adhesive for medicinal purpose prepared by laminating a film of a polyion complex formed of a polycationic substance and a polyanionic substance. Moreover, Non-Patent Literature 1 discloses a bioadhesive thin film prepared by laminating chitosan and alginic acid alternately.

### Citation List

### Patent Literature

Patent Literature 1: Japanese Patent No. 4241985

### Non Patent Literature

Non-Patent Literature 1: T.Fujie et al., Adv. Funct. Mater., 2009, volume 19, pages 2560-2568

### Summary of Invention

### Problems to be Solved by the Invention

The bioadhesive thin film disclosed in Non-Patent Literature 1 is produced by alternately laminating chitosan and alginate, which have good track record as medical materials and are polysaccharides having high biocompatibility and high biodegradability, and therefore, the bioadhesive thin film excels in toughness and transparency and has high adhesion. Furthermore, the bioadhesive thin film has an extremely thin thickness of about 75 nm and in the case where a tender living tissue such as the lung is damaged, it is possible to stably occlude the tender living tissue even if a load of about sneezing is applied only by attaching the bioadhesive thin film thereto.

However, the bioadhesive thin film disclosed in Non-Patent Literature 1 is produced by a spin coating method. Therefore, there is a need to use a lot of drug solutions at the time of production and every single laminated layer must be formed, and hence, there are problems in economic efficiency and mass productivity, resulting in difficulty in production on an industrial scale and low general versatility.

Thus, an object of the present invention is to provide a thin film with a base which can be used widely and a method for producing the same.

### Means for Solving the Problems

The present invention provides a thin film with a base comprising a base and a thin film formed on the base, wherein the thin film comprises a layer A formed by using a solution containing a polycation and a layer B formed by using a solution containing a polyanion and having a pH of 1.6 to 5.4.

The thin film with a base of the present invention can be easily produced on an industrial scale and can be used widely because of the above described constitution. Moreover, when the thin film is attached to an affected part, the thin film adheres to the affected part with high adhesion to a tissue and has sufficient strength as a tissue adhesive (bioadhesive thin film). Furthermore, since the thin film with a base has less danger of infection or the like and excellent safety for a living organism, it is possible to use the thin film as a tissue adhesive easily and effectively. Furthermore, the thin film with a base does not need pretreatment such as dissolution and has extremely excellent handleability.

It is preferred that the solution containing a polyanion and having a pH of 1.6 to 5.4 is a solution containing a polyanion and malic acid. By containing malic acid, it is possible to stably maintain the pH of the solution and the production efficiency of the thin film is improved.

It is preferred that the thin film is a film in which the layer A and the layer B are laminated alternately. By laminating the layer A and the layer B alternately, a thin film having higher mechanical strength and higher self-adhesion is obtained. It is noted that alternately laminating the layer A and the layer B includes not only a case where a single layer A and a single layer B are laminated alternately but also a case where a plurality of layers A and a plurality of layers B are laminated alternately.

The polycation is preferably a cationic polymer having two or more amino groups in one molecule, more preferably a basic polysaccharide or a derivative thereof, or a salt thereof, especially preferably chitosan or a derivative thereof, or a salt thereof. By this, it is possible to obtain a thin film having higher bioabsorbability.

The polyanion is preferably an anionic polymer having two or more carboxyl groups or carboxylate groups in one molecule, more preferably an acidic polysaccharide or a derivative thereof, or a salt thereof, still more preferably alginic acid or a derivative thereof, or a salt thereof. By this, it is possible to obtain a thin film having higher biocompatibility.

Also, the present invention provides a method for producing a thin film with a base, comprising a layer formation step of allowing a base to come into contact with a solution containing a polycation or a solution containing a polyanion and having a pH of 1.6 to 5.4 to form a layer derived from the polycation or the polyanion on the surface of the base; and a laminating step of repeating
a step (i) of allowing a solution containing a polyanion and having a pH of 1.6 to 5.4 to come into contact with the layer derived from the polycation to form a layer derived from the polyanion on the layer derived from the polycation, and
a step (ii) of allowing a solution containing a polycation to come into contact with the layer derived from the polyanion to form a layer derived from the polycation on the layer derived from the polyanion.

It is possible to produce a thin film with a base easily and rapidly by the production method of the present invention because of the above described constitution. Moreover, the production method allows easy production on an industrial scale and can be used widely.

In the above described production method, it is preferred to use a solution containing a polyanion and malic acid as the solution containing a polyanion and having a pH of 1.6 to 5.4. By using the solution containing a polyanion and malic acid, the production efficiency is more improved.

In the above described production method, it is preferred to repeat the laminating step until the numbers of the layer derived from the polycation and the layer derived from the polyanion are both 1 to 300. By this, the transparency of the thin film can be easily maintained.

### Effects of the Invention

According to the present invention, a thin film with a base which can be used widely and a method for producing the same are provided. Specifically, the thin film according to the present invention is suitable for use as an adhesive which can be attached to a cell, a tissue, an organ, a blood vessel wall, a mucous membrane, a cornea, skin, hair, a nail, or the like.

### Brief Description of Drawings

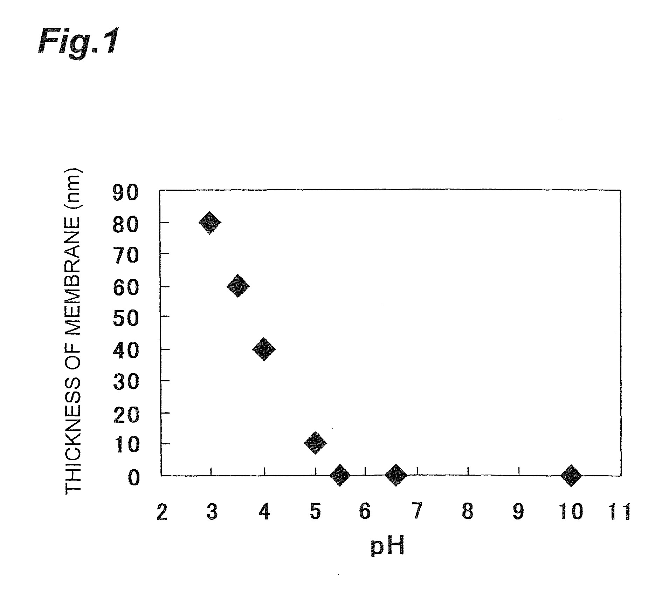

[Figure 1] Figure 1 is a plot of the pH of an anionic polymer aqueous solution vs. the thickness of a laminated membrane in the case of alternately laminating a cationic polymer and an anionic polymer on a SiO₂ substrate (cycle: 30 times).

### Embodiments for Carrying Out the Invention

### [Polycation]

In the present specification, the polycation means a compound having two or more cationic groups in one molecule, and the cationic group means a cation group or a group which can be converted to a cation group. Examples of the cationic group include an amino group; a monoalkylamino group such as a methylamino group and an ethylamino group; a dialkylamino group such as a dimethylamino group and a diethylamino group; an imino group; and a guanidino group. It is noted that the amino group may be -NH₃⁺, in which a proton binds via a coordinate bond.

As the polycation, a cationic polymer is preferred. It is noted that, in the present specification, the cationic polymer means a polymer having two or more cationic groups in one molecule. As the cationic polymer, one prepared by polymerizing a monomer having a cationic group is preferred.

As the cationic polymer, one which can form a gel-like polyion complex with an anionic polymer described below in the presence of water, can provide the polyion complex having adhesion action to a living tissue, and is non-toxic to a living organism is preferred. Moreover, as the cationic polymer, a substance having bioabsorbability is preferred such that the substance is biodegraded and then absorbed in a living organism after a tissue of an affected area is cured.

As the cationic polymer, it is preferred to use a polymer which has such hydrophilicity that the polymer can be dissolved in or swollen with an acidic aqueous solution, and in the acidic aqueous solution, has a property of having a positive electric charge resulting from a bond between the cationic group and a proton. As the cationic polymer, a polymer having two or more amino groups in one molecule is preferred.

Preferred examples of the cationic polymer include a basic polysaccharide such as collagen, polyhistidine, ionene, chitosan, and aminated cellulose; a homopolymer and a copolymer of a basic amino acid such as polylysine, polyarginine, and a copolymer of lysine and arginine; a basic vinylpolymer such as polyvinylamine, polyallylamine, and polydivinylpyridine; and a salt thereof (a hydrochloride salt, an acetate salt, or the like); polyethyleneimine, polyallylamine hydrochloride, and poly diallyldimethylammonium chloride.

Also, a cross-linked polymer obtained by cross-linking the above described cationic polymer may be used. As the method of cross-linking the cationic polymer, any of known methods can be used. When the cationic polymer has an amino group, a method of cross-linking by a condensation reaction between the amino group in the cationic polymer and dicarboxylic acid is preferred.

As the cationic polymer, the basic polysaccharide or a derivative thereof (for example, an acetylate or the like), or a salt thereof is preferred. As the basic polysaccharide, chitosan is especially preferred. Chitosan is a deacetylate of chitin, and the deacetylation degree preferably ranges from 40 to 100%, more preferably ranges from 45 to 90%, still more preferably ranges from 50 to 80% because the bioabsorbability and the water solubility become higher.

The molecular weight of the cationic polymer is not particularly limited, but the viscosity-average molecular weight of the cationic polymer preferably ranges from 1,000 to 500,000, more preferably ranges from 10,000 to 400,000, still more preferably ranges from 50,000 to 200,000 because, when the viscosity-average molecular weight is higher, the viscosity of a solution becomes higher at the time of production of the thin film with a base, and then, casting tends to be difficult, and bioabsorbability tends to be decreased.

In the present specification, "viscosity-average molecular weight" may be evaluated by viscometry, which is a general measuring method, and for example, Mv may be calculated based on an intrinsic-viscosity number [η] measured in accordance with JIS K 7367-3: 1999.

As the polycation, even a low molecular compound having two or more cationic groups in one molecule can be preferably used. Examples of the low molecular compound having two or more cationic groups in one molecule include low molecular diamine and polyamine. Specific examples include a compound having two amino groups in one molecule such as a diaminoalkane including diaminoethane, diaminopropane, diaminobutane, diaminopentane, diaminohexane, and the like; a compound having three to four amino groups in one molecule such as a mono or di lysylaminoalkane including N-(lysyl)-diaminoethane, N,N'-(dilysyl)-diaminoethane, N-(lysyl)-diaminohexane, N,N'-(dilysyl)-diaminohexane, and the like; and a compound having five or more amino groups in one molecule.

### [Solution containing polycation]

In a solution containing the polycation, the concentration of the polycation is preferably 0.01 to 5.0 mass%, more preferably 0.02 to 2.0 mass%, especially preferably 0.05 to 1.0 mass%.

The viscosity of the solution containing the polycation preferably ranges from 0.1 to 1000 mPa·s, more preferably ranges from 0.5 to 500 mPa·s, still more preferably ranges from 1 to 100 mPa·s. In the present specification, the viscosity means a value measured by using a tuning fork vibro viscometer SV-10 manufactured by A&D Company, Limited and using 10 mL of a sample at 20 °C.

In the solution containing the polycation, two or more types of polycation may be used in combination.

As a solvent used for the solution containing the polycation, any solvent can be used as long as the polycation can be dissolved in the solvent, and water or an aqueous solution of an inorganic salt is appropriate because the charge amount of the polycation can be increased.

It is not necessary to adjust the pH of the solution containing the polycation, and it is possible to directly use a solution prepared by dissolving the polycation in the solvent. For example, it is possible to set the pH to 1.2 to 6.6.

### [Polyanion]

In the present specification, the polyanion means a compound having two or more anionic groups in one molecule, and the anionic group means an anion group or a group which can be converted to an anion group. Examples of the anionic group include a carboxyl group, a carboxylate group, a sulfate group, a sulphonate group, and a phosphate group.

As the polyanion, an anionic polymer is preferred. It is noted that, in the present specification, the anionic polymer means a polymer having two or more anionic groups in one molecule. The anionic polymer is preferably one prepared by polymerizing a monomer having an anionic group.

As the anionic polymer, one which can form the gel-like polyion complex with the cationic polymer described above in the presence of water, can provide the polyion complex having adhesion action to a living tissue, and has less toxic reaction to a living organism is preferred. Moreover, as the anionic polymer, a substance having bioabsorbability is preferred such that the substance is biodegraded and then absorbed in a living organism after a tissue of an affected area is cured.

As the anionic polymer, a polymer which has such hydrophilicity that the polymer can be dissolved in or swollen with water, and in water, has a property of having a negative electric charge resulting from dissociation of a proton or a metal ion in the anionic group is preferably used. As the anionic polymer, a polymer having two or more carboxyl groups or carboxylate groups in one molecule is especially preferred.

Preferred examples of the anionic polymer include a natural acidic polysaccharide having an anionic group such as a carboxyl group, a carboxylate group, or a sulfate group, including alginic acid, hyaluronic acid, chondroitin sulphuric acid, dextran sulphuric acid, pectin, and sacran, and a derivative thereof; an acidic polysaccharide artificially synthesized by binding an anionic group to a polysaccharide which naturally does not have an anionic group such as a carboxyl group, a carboxylate group, or a sulfate group, including cellulose, dextran, and starch, and a derivative thereof (for example, carboxymethylcellulose, carboxymethyldextran, carboxymethylstarch, carboxymethylchitosan, sulfated cellulose and sulfated dextran, and a derivative thereof); a homopolymer and a copolymer of an acidic amino acid such as polyglutamic acid, polyasparagine acid, and a copolymer of glutamic acid and asparagine acid; an acidic vinyl polymer such as polyacrylic acid; and a salt thereof (for example, an alkali metal salt such as a sodium salt).

Examples of the derivative of the acidic polysaccharide include a compound obtained by reacting the whole or a part of hydroxyl group with acetic acid, nitric acid, sulphuric acid, phosphoric acid, or the like; and a compound obtained by esterifying a part of carboxyl group or carboxylate group with a low molecular alcohol such as ethylene glycol and propylene glycol.

Specific examples of the derivative of the acidic polysaccharide include alginic acid ethylene glycol ester, alginic acid propylene glycol ester, hyaluronic acid ethylene glycol ester, and hyaluronic acid propylene glycol ester. The esterification degree of the derivative is not particularly limited, but when the esterification degree becomes too high, the ratio of the carboxyl group or the carboxylate group, that is, the anionic property decreases, and then, the mechanical strength of the polyion complex which is to be formed with the cationic polymer tends to decrease. Hence, the esterification degree of the derivative preferably ranges from 40 to 100%, more preferably ranges from 45 to 90%, still more preferably ranges from 50 to 80%.

Examples of the salt of the acidic polysaccharide or the derivative of the acidic polysaccharide include a salt of the acidic polysaccharide or the derivative of the acidic polysaccharide and a monovalent ion, such as an alkali metal salt including a sodium salt, a potassium salt, and the like; and an ammonium salt.

Also, it is possible to use a cross-linked polymer obtained by cross-linking the above described anionic polymer. As the method of cross-linking the anionic polymer, any of known methods can be used. When the anionic polymer has a carboxyl group or a carboxylate group, a method of cross-linking by a condensation reaction of the carboxyl group or the carboxylate group in the anionic polymer with diamine is preferred.

As the anionic polymer, the acidic polysaccharide or the derivative thereof, or the salt thereof is preferred. Especially, alginic acid or the derivative thereof (specifically, alginic acid propylene glycol ester or the like), or the salt thereof (for example, an alkali metal salt such as a sodium salt) is preferred because such compound is a natural polysaccharide, has excellent biocompatibility, and is easily available.

The molecular weight of the anionic polymer is not particularly limited, but the viscosity-average molecular weight of the anionic polymer preferably ranges from 1,000 to 500,000, more preferably ranges from 10,000 to 400,000, still more preferably ranges from 50,000 to 200,000 because, when the viscosity-average molecular weight becomes higher, the viscosity of a solution becomes higher at the time of production of the thin film with a base, and then, casting tends to be difficult, and the bioabsorbability tends to be decreased.

As the polyanion, even a low molecular compound having two or more anionic groups in one molecule can be preferably used. Examples of the low molecular compound having two or more anionic groups in one molecule include a compound having two carboxyl groups or carboxylate groups in one molecule such as succinic acid and malonic acid.

The combination of the cationic polymer and the anionic polymer may be any combination as long as the cationic polymer and the anionic polymer form the polyion complex and become gelled when mixed under the presence of water. Especially, a combination employing a bioabsorbable polymer as at least one of the cationic polymer and the anionic polymer is preferred because of higher safety.

The bioabsorbable polymer means a polymer which can be biodegraded. Specific examples of such cationic polymer include chitosan, collagen, polylysine, polyarginine, polyhistidine, and ionene, and specific examples of such anionic polymer include alginic acid, hyaluronic acid, polyglutamic acid, chondroitin sulphuric acid and a derivative thereof.

### [Solution containing polyanion and having pH of 1.6 to 5.4]

In a solution containing the polyanion and having a pH of 1.6 to 5.4 (hereinafter, also referred to as "solution containing the polyanion"), the concentration of the polyanion is preferably 0.01 to 5.0 mass%, more preferably 0.02 to 2 mass%, especially preferably 0.05 to 1.0 mass%.

The viscosity of the solution containing the polyanion preferably ranges from 0.1 to 1000 mPa·s, more preferably ranges from 1 to 500 mPa·s, still more preferably ranges from 10 to 100 mPa·s.

The pH of the solution containing the polyanion is 1.6 to 5.4; however, the pH preferably ranges from 1.8 to 5.0, more preferably ranges from 2.0 to 4.5, especially preferably ranges from 2.5 to 4.0 because the solubility of the polyanion becomes higher.

The pH of the solution containing the polyanion can be adjusted by adding an acidic component. Examples of the acidic component include an organic acid such as acetic acid, propionic acid, succinic acid, malonic acid, oxalic acid, and malic acid; and an inorganic acid such as hydrochloric acid, sulphuric acid, and nitric acid.

In the solution containing the polyanion, two or more types of polyanions may be used in combination.

As a solvent used for the solution containing the polyanion, any solvent can be used as long as the polyanion can be dissolved in the solvent, and water or an aqueous solution of an inorganic salt is appropriate because the charge amount of the polyanion can be increased.

The solution containing the polyanion is preferably a solution containing the polyanion and malic acid (in the present specification, also referred to as "solution C"). It is possible to obtain a solution having more excellent handling property because malic acid has less irritating odor. Moreover, it is possible to maintain the pH of the solution more stably because malic acid is less volatile. The content of malic acid in the solution C can be appropriately adjusted depending on the type of the polyanion, but for example, the content is preferably 0.5 to 100 mass parts, more preferably 1 to 50 mass parts, especially preferably 5 to 20 mass parts relative to 1 mass part of the polyanion.

It is possible to add an acidic component other than malic acid to the solution C within a scope which does not impair the effect of the present invention. Examples of the acidic component other than malic acid include an organic acid such as oxalic acid, citric acid, gluconic acid, succinic acid, tartaric acid, fumaric acid, malic acid, pyrophosphoric acid, lactic acid, and benzoic acid; an inorganic acid such as hydrogen fluoride, hydrogen peroxide, carbonic acid, hydrochloric acid, perchloric acid, nitric acid, sulphuric acid, sulphurous acid, persulphuric acid, phosphoric acid, phosphorous acid, and hypophosphorous acid; and an organic acid having these as a functional group.

The amount of the acidic component other than malic acid added to the solution C preferably ranges from 0.01 to 10 mass%, more preferably ranges from 0.05 to 8 mass%, still more preferably ranges from 0.1 to 6 mass% relative to the total amount of the solution C.

### [Base]

A base serves as a support substrate at the time of production of the thin film with a base. Examples of a material usable as the base include a resin, a semiconductor such as silicone, a metal, ceramics, glass, paper, non-woven fabrics, an inorganic non-metal, a woody material, and a powder. It is possible to set the shape of the base to an arbitral shape such as a film shape, a sheet shape, a plate shape, and a shape with a curved surface. Among these, a resin film having flexibility is preferred in consideration of the mass productivity.

In the case of using the resin film having flexibility, the thickness of the resin film is not particularly limited, but is preferably 5 µm to 500 µm, more preferably 25 to 250 µm in view of practical use.

The resin of the resin film may be either thermoplastic resin or thermosetting resin, and examples thereof include a polyolefin such as polyethylene (high density, medium density, or low density), polypropylene (isotactic type or syndiotactic type), polybutene, an ethylene-propylene copolymer, an ethylene-vinyl acetate copolymer (EVA), and an ethylene-propylene-butene copolymer; a cyclic polyolefin, a modified polyolefin, polyvinyl chloride, polyvinylidene chloride, polystyrene, polyamide, polyimide, polyamideimide, polycarbonate, poly-(4-methylpentene-1), an ionomer, an acrylic resin, polymethyl methacrylate, polybutyl (meth)acrylate, a methyl (meth)acrylate-butyl (meth)acrylate copolymer, a methyl (meth)acrylate-styrene copolymer, an acrylic-styrene copolymer (AS resin), a butadiene-styrene copolymer, an ethylene-vinyl alcohol copolymer (EVOH); a polyester such as polyethylene terephthalate (PET), polybutylene terephthalate (PBT), an ethylene-terephthalate-isophthalate copolymer, polyethylene naphthalate, and polycyclohexane terephthalate (PCT); polyether, polyether ketone (PEK), polyether ether ketone (PEEK), polyetherimide, polyacetal (POM), polyphenylene oxide, modified polyphenylene oxide, polyarylate, aromatic polyester (liquid crystal polymer), polytetrafluoroethylene, polyvinylidene fluoride, and other fluorine resins; a thermoplastic elastomer such as styrene based, polyolefin based, polyvinyl chloride based, polyurethane based, fluorine rubber based, and chlorinated polyethylene based; an epoxy resin, a phenol resin, an urea resin, a melamine resin, unsaturated polyester, a silicone resin, polyurethane, and nylon; a cellulose resin such as nitrocellulose, cellulose acetate, and cellulose acetate propionate; and a copolymer, a blend, and a polymer alloy mainly composed thereof. These can be used alone or in combination of two or more (for example, as a laminate composed of two or more layers).

Among these resin films, the polyethylene terephthalate (PET) film is more preferred because the adhesion of a laminated membrane is more excellent.

Examples of the glass include silicate glass (quartz glass), alkali silicate glass, soda lime glass, potash lime glass, lead (alkali) glass, barium glass, and borosilicate glass.

Examples of the metal include gold, chrome, silver, copper, iron, titanium, nickel, tungsten, tantalum, aluminum, and platinum. Also, it is possible to use an alloy of above described metal, such as stainless steel including SUS316L and the like, a shape-memory alloy including a Ti-Ni alloy, a Cu-Al-Mn alloy, and the like, a Cu-Zn alloy, a Ni-Al alloy, a titanium alloy, a tantalum alloy, a platinum alloy, and a tungsten alloy.

Examples of the ceramics include an oxide (for example, aluminum oxide, zinc oxide, titanium oxide, silicon oxide, zirconia, and barium titanate), a nitride (for example, silicon nitride and boron nitride), a sulfide (for example, cadmium sulfide), and a carbide (for example, silicon carbide). Also, it is possible to use a mixture thereof.

Examples of the paper include tissue paper, kraft paper, high-quality paper, linter paper, baryta paper, parchment paper, and Japan paper.

Examples of the non-woven fabrics include non-woven fabrics composed of a fiber such as a polyester resin, an acrylic resin, nylon, vinylon, and glass. The paper or the non-woven fabrics may be one in which the strength between the fibers or interlayer strength with another layer is enhanced. Moreover, one in which a resin such as an acrylic resin, a styrene butadiene rubber, a melamine resin, and an urethane resin is added (impregnated after making paper or internally filled at the time of making paper) may be used in view of suppressing scuffing or reducing permeability.

Examples of the inorganic non-metal include an inorganic material such as a non-ceramic material including sheet-forming cement, extrusion cement, slag cement, ALC (autoclaved lightweight concrete), GRC (glass fiber reinforced concrete), pulp cement, wood chip cement, asbestos cement, calcium silicate, gypsum, gypsum slag, and the like; and ceramics including earthenware, pottery, porcelain, stoneware, glass, enamel, and the like.

Examples of the woody material include a single board, a plyboard, a particle board, a fiber board, and bonded wood composed of Japanese cedar, Japanese cypress, oak, lauan, teak, or the like.

Examples of the powder include an inorganic pigment such as iron oxide, zinc oxide, cerium oxide, magnesium oxide, zirconium oxide, plate-like aluminum oxide, barium sulfate, chrome oxide, ultramarine, magnesium carbonate, calcium carbonate, mica, synthetic mica, sericite, talc, silica, plate-like silica, kaolin, sillimanite, chrome hydroxide, zinc oxide, carbon black, alumina, aluminum silicate, magnesium silicate, boron nitride, a silica-alumina powder, bentonite, smectite, magnesium fluoride, and hydroxylapatite; an organic powder such as a nylon powder, polymethyl methacrylate, a styrene-divinylbenzene copolymer, a polyethylene powder, a silicone resin, a Teflon (registered trademark) powder, a silicone gum, a silk powder, carnauba wax, rice wax, starch, and micro crystalline cellulose; an organic dye such as rhodamine B; an organic colorant such as a zirconium, barium, or aluminum lake of red No. 201, black No. 401, yellow No. 4, blue No. 1, or the like; a composite powder such as titanated mica and mica coated with iron oxide; and a powder with surface treatment. As the shape of the powder, any shape and any particle size ordinarily used for a cosmetic material may be employed, such as a spherical shape, a plate-like shape, a needle-like shape, and a fibrous shape. A preferred powder is the inorganic pigment.

Moreover, it is possible to subject the surface of the base to corona discharge treatment, glow discharge treatment, plasma treatment, ultraviolet irradiation treatment, ozone treatment, chemical etching treatment using an alkali or an acid, or the like.

The base may have a resin membrane, an inorganic membrane, or a membrane including an organic material and an inorganic material (organic-inorganic membrane) laminated thereon. A laminate structure composed of the resin membrane layer, the inorganic membrane layer, or the organic-inorganic membrane layer may cover a part of the surface of the base. Moreover, in the laminate structure, a membrane which is not the outermost layer does not have to have a polar group.

### [Thin film]

The thin film of the present embodiment comprises a layer A formed by using the solution containing the polycation and a layer B formed by using the solution containing the polyanion and having a pH of 1.6 to 5.4. Moreover, the thin film of the present embodiment is preferably an alternately laminated thin film in which the layer A and the layer B are alternately laminated.

In the case of employing a film in which the layer A and the layer B are alternately laminated, the number of laminated layers is not particularly limited, but the numbers of the layer A and the layer B are both preferably 1 to 300 because the transparency of the thin film tends to be maintained easily. Furthermore, the numbers of the layer A and the layer B are both more preferably 10 to 100, especially preferably 20 to 80 because the thin film tends to have such a thickness that the thin film has self-adhesion.

A laminate structure composed of the layer A and the layer B in the thin film of the present embodiment can be identified by, for example, observing the thin film by IR, NMR, TOF-SIMS, or the like.

The thickness of the thin film of the present embodiment is not particularly limited, but preferably ranges from 1 nm to 300 nm, more preferably ranges from 10 nm to 250 nm, still more preferably ranges from 20 nm to 200 nm because properties such as self-adhesion, water absorbability, and flexibility in a dry state become more excellent.

The thin film of the present embodiment can be also used as a drug carrier (for example, a functional support or substitute for platelet in a drug delivery system). When the thin film is used as the drug carrier, the thin film may be modified with a functional substance such as (a) a drug, (b) a substance containing a site specifically recognizing a target tissue/cell (specific recognition substance), or (c) a substance for stabilizing a structure in a body (stabilization substance). Specific examples of these functional substances are follows.
(a) Drug: anti-inflammatory agent, hemostatic agent, vasodilatory drug, thrombolytic agent, anti-arteriosclerotic agent, or the like
(b) Specific recognition substance: collagen, laminin, VCAM-1, selectin, fibrin, or the like
(c) Substance for stabilizing a structure: polyethylene glycol, polyvinylpyrrolidone, polyvinylalcohol, polysaccharide, polyglutamic acid, or the like

An example of a method for modifying the thin film with the functional substance is a method of allowing a functional group of the functional substance and a functional group of the thin film to form a chemical bond. Examples of such method include, in the case where the functional substance has a hydroxyl group or an amino group and the thin film has an isocyanato group, a method of allowing the functional groups to form an urethane bond or an urea bond; in the case where the functional substance has a carboxyl group and the thin film has an amino group, a method of activating the carboxyl group to form an amide bond with the amino group; in the case where both of the functional substance and the thin film have an amino group, a method of allowing the amino groups to bind to each other via Schiff's base formed with glutaraldehyde; in the case where the functional substance has a carboxyl group and the thin film has an amino group or a hydroxyl group, a method of allowing the carboxyl group and the amino group or the hydroxyl group to form an amide bond or an ester bond; in the case where the functional substance is polysaccharide and the thin film has an amino group, a method of allowing a hydroxyl group of the functional substance to form imide carbonate with cyan bromide and then cross-linking it with the amino group; and in the case where both of the functional substance and the thin film have a mercapto group, a method of allowing the activated mercapto groups to form a disulfide bond.

Moreover, an alkyl diimidate, an acyl diazide, a diisocyanate, a bismaleimide, a triazinyl, a diazo compound, glutaraldehyde, N-succinimidyl-3-(2-pyridyldithio)alkyonate, bromocyan, or the like is used as a cross-linking agent and corresponding groups in the functional substance and the thin film may be cross-linked.

Furthermore, in the case where the functional substance is hydrophobic, a method of allowing the functional substance to bind to a hydrophobic area of the thin film by hydrophobic interaction may be used. In the case where the functional substance has a hydrogen bonding property, a method of allowing the functional substance to bind to a hydrogen bonding area of the thin film by a hydrogen bonding may be used. In the case where the functional substance has a charge, a method of allowing the functional substance to bind to an opposite charge area of the thin film by electrostatic interaction may be used.

The thin film of the present embodiment has a bioadhesive property, and is especially suitable for use as an adhesive having a sheet shape. For example, the thin film of the present embodiment is used for adhesion of a cell, a tissue, an organ, a blood vessel wall, a mucous membrane, a cornea, skin, hair, a nail, or skin; for adhesion of an incision in a parenchyma organ such as the liver and the spleen; for anastomosis of the intestinal tract, the fallopian tube, or the like; for adhesion of a membrane such as dura mater, pleura, fascia, and peritoneum; as a hemostatic adhesive for stopping oozing bleed from a parenchyma organ; as a suture subsidiary material for stopping bleeding or the like from a suture hole at the time of saturation; and as an adhesive for suppressing air leak from the lung.

### [Method for producing thin film with base]

For example, the thin film with a base of the present embodiment can be produced from the base, the solution containing a polycation (hereinafter, also referred to as "solution A"), and a solution containing a polyanion and having a pH of 1.6 to 5.4 (hereinafter, also referred to as "solution B") by alternate lamination disclosed in Langmuir, vol. 13, pp. 6195-6203 (1997).

Specifically, the method for producing the thin film with a base of the present embodiment comprises a layer formation step of allowing the base to come into contact with the solution A or the solution B to form a layer derived from the polycation or the polyanion on the surface of the base; and a laminating step of repeating
a step (i) of allowing the solution B to come into contact with the layer derived from the polycation to form a layer derived from the polyanion on the layer derived from the polycation, and
a step (ii) of allowing the solution A to come into contact with the layer derived from the polyanion to form a layer derived from the polycation on the layer derived from the polyanion.

By this alternate lamination, the layer derived from the polycation (or the layer derived from the polyanion) formed on the base and the solution B (or the solution A) come into contact with each other, and then, the polycation and the polyanion are adsorbed alternately to form a laminated membrane. Moreover, when the adsorption of the polycation or the polyanion proceeds by the contact and the charge of the surface is inverted, further electrostatic adsorption does not occur, and therefore, the thickness of the layer formed by the contact with the solution A or the solution B can be controlled.

In the layer formation step, the layer derived from the polycation is formed on the surface of the base by allowing the base to come into contact with the solution A, or the layer derived from the polyanion is formed on the surface of the base by allowing the base to come into contact with the solution B. When the surface of the base is negatively charged, it is preferred to conduct the former, and when the surface of the base is positively charged, it is preferred to conduct the latter. At least a part of the surface of the base may be allowed to come into contact with the solution A or the solution B. The contact with the solution A or the solution B may be conducted in two or more installments.

In the laminating step, the charge of the surface may be inverted in the step (i) or the step (ii). The frequency of the contact is not particularly limited. For example, the contact with the solution B may be conducted in two or more installments in the step (i), and the contact with the solution A may be conducted in two or more installments in the step (ii).

The repeating time of the step (i) or the step (ii) in the laminating step is not particularly limited, but it is preferred to repeat those steps until the numbers of the layer derived from the polycation and the layer derived from the polyanion are both 1 to 300 because the transparency of the thin film tends to be maintained easily. Moreover, it is more preferred to repeat the steps until the numbers of the layer derived from the polycation and the layer derived from the polyanion are both 10 to 100, especially preferred to repeat the steps until the numbers of the layers are both 20 to 80, because the thin film tends to have such a thickness that the thin film has self-adhesion. It is noted that the thickness of the thin film can be controlled by controlling the repeating time in the laminating step.

In the production method, the laminating step preferably ends with the step (ii) rather than with the step (i). By this, the properties of the substance used as the polycation can be exerted more easily. For example, in the case of using chitosan as the polycation, an antibiotic property, which is a property of chitosan, can be exerted more easily.

In the production method, it is preferred to rinsing an adsorbed surface after contact with the solution A or the solution B in the layer formation step or the laminating step. By this, it is possible to remove an extra material from the adsorbed surface.

Preferred examples of a rinse solution used for the rinse include water, an organic solvent, and a mixed solvent of water and a water-soluble organic solvent. Examples of the water-soluble organic solvent include methanol, ethanol, propanol, acetone, dimethylformamide, and acetonitrile.

In the production method, it is preferred to immerse the base, the layer derived from the polycation, or the layer derived from the polyanion into the solution A or the solution B to achieve the contact. For example, in the layer formation step, it is preferred to immerse the base into the solution A or the solution B to achieve the contact, and in the laminating step, it is preferred to immerse the layer derived from the polycation (or the layer derived from the polyanion) into the solution B (or the solution A) to achieve the contact. By this, production on an industrial scale is more easily achieved and the production method can be used more widely.

As a device for forming the laminated membrane, a device called a dipper disclosed in J. Appl. Phys., Vol. 79, pp. 7501-7509, (1996) and Japanese Patent Application No. 2000-568599 may be used. In the case of using a dipper, an arm having the base fixed thereon moves automatically, and the base can be immersed in the solution A, the solution B, or the rinse solution sequentially according to a program.

By employing a method of alternately immersing the object (hereinafter, also referred to as "alternate immersion method"), it is possible to continue formation of the layers as long as the surface charge is inverted. Therefore, the uniformity of the thickness and thickness controllability of the thin film formed by the alternate immersion method is higher than those of a film formed by a conventional dip coating method.

The alternate immersion method can be employed for a base even if the whole or a part of which has a tubular shape, a thread-like shape, a fibrous shape, a foam-like shape, or the like as long as the solution can penetrate into the base by immersion because the laminated membrane is formed on the surface. Moreover, even if the surface of the base has a concave-convex shape, the laminated membrane can be formed by following the surface structure. Furthermore, even if the surface of the base has a nanometer-scale or submicron-scale structure, the laminated membrane can be formed by following the structure.

The thin film with a base of the present embodiment may be produced by forming the laminated membrane by a spin coating method in which the solution A or the solution B is dropped or sprayed on the base. In this case, the rinse solution may be provided by dropping, spraying, shower, or a combination thereof. The base may conduct a movement such as transportation and rotation. However, the spin coating method has a disadvantage in that the mass productivity is poor because the amount of the solution A, the solution B, or the like is large and every single layer must be formed.

In each case of employing the above described production methods, as a solvent used for the solution A or the solution B, any solvent can be arbitrarily used as long as the solvent can dissolve the polycation or the polyanion, but water or a aqueous solution of an inorganic salt is appropriate because the charge amount of the polycation or the polyanion can be more increased. The concentration of the polycation or the polyanion in the solution is not particularly limited and may be arbitrarily set depending on the production method.

Furthermore, in the case where at least one of the polycation and the polyanion is a salt and water solubility of the polycation or the polyanion is decreased by removing a counter ion of the cation group or the anion group in the salt, it is possible to improve the mechanical strength of the thin film by removing the counter ion contained in the thin film after forming the thin film with a base. Removal of the counter ion can be conducted by, for example, increasing the frequency of a cleaning step, immersion into a pH adjusting solution, or the like.

It is noted that, in the production method, a solution containing the polyanion and malic acid (the solution C) is preferably used as the solution B.

### Examples

Hereinafter, the present invention will be more specifically described based on Examples and Comparative Examples, which by no means limit the present invention.

A chitosan aqueous solution (manufactured by KIMICA corporation: viscosity average molecular weight 90,000, viscosity 12.5 mPa·s, concentration: 0.1 mass%) was used as the cationic polymer, and a sodium alginate aqueous solution (manufactured by KIMICA corporation: viscosity average molecular weight 100,000, viscosity 6.7 mPa·s, concentration: 0.1 mass%) was used as the anionic polymer. In Examples 1 to 5 and Comparative Examples 1 to 3, acetic acid (manufactured by Wako Pure Chemical Industries, Ltd.), hydrochloric acid (manufactured by Wako Pure Chemical Industries, Ltd.), or a mixed basic buffer solution of sodium hydrogen carbonate (manufactured by Wako Pure Chemical Industries, Ltd.) and sodium carbonate (manufactured by Wako Pure Chemical Industries, Ltd.) was used as the acidic component. In Examples 6 to 9 and Comparative Examples 4 and 5, malic acid (manufactured by Wako Pure Chemical Industries, Ltd.), acetic acid (manufactured by Wako Pure Chemical Industries, Ltd.), hydrochloric acid (manufactured by Wako Pure Chemical Industries, Ltd.), or nitric acid (manufactured by Wako Pure Chemical Industries, Ltd.) was used as the acidic component. For measurement of the pH of the solution, a pH meter D-50 (manufactured by HORIBA, Ltd.) was used.

### [Example 1]

As the chitosan aqueous solution, the above described 0.1 mass% chitosan aqueous solution was directly used. As the sodium alginate aqueous solution, one obtained by dropping acetic acid in the above described 0.1 mass% sodium alginate aqueous solution to adjust the pH to 3.0 was used.

A SiO₂ substrate (manufactured by ASAHI SANGYO KAISHA, Ltd., 5 inch silicon wafer: 30 mm x 70 mm x 1.0 mm thickness) was (i) immersed into the chitosan aqueous solution for 1 minute and then immersed into ultrapure water for rinse (specific resistance 18 MΩ·cm) for 1 minute, and (ii) immersed into the sodium alginate aqueous solution for 1 minute and then immersed into ultrapure water for rinse for 1 minute.

A procedure of repeating (i) and (ii) in order is taken as 1 cycle, and the cycle was repeated 30 times, thus obtaining a laminated membrane of chitosan and sodium alginate on the SiO₂ substrate. The thickness of the obtained laminated membrane was measured by an ellipsometer (manufactured by Mizojiri Optical Co., Ltd., light source 633 nm). The result was that the thickness was 80 nm.

### [Example 2]

The same process as that in Example 1 was conducted except for one obtained by dropping acetic acid in the 0.1 mass% sodium alginate aqueous solution to adjust the pH to 3.5 was used as the sodium alginate aqueous solution. The thickness of the obtained laminated membrane was 60 nm.

### [Example 3]

The same process as that in Example 1 was conducted except for one obtained by dropping acetic acid in the 0.1 mass% sodium alginate aqueous solution to adjust the pH to 4.0 was used as the sodium alginate aqueous solution. The thickness of the obtained laminated membrane was 40 nm.

### [Example 4]

The same process as that in Example 1 was conducted except for one obtained by dropping acetic acid in the 0.1 mass% sodium alginate aqueous solution to adjust the pH to 5.0 was used as the sodium alginate aqueous solution. The thickness of the obtained laminated membrane was 10 nm.

### [Example 5]

The same process as that in Example 1 was conducted except for PET (manufactured by TOYOBO CO., LTD., A4100, thickness: 125 µm) was used as the base instead of the SiO₂ substrate. The thickness of the obtained laminated membrane was 85 nm.

In the case of using PET as the base, it was possible to increase the thickness in comparison with the case of using the SiO₂ substrate.

### [Comparative Example 1]

The same process as that in Example 1 was conducted except for the pH adjusting agent was not added and the 0.1 mass% sodium alginate aqueous solution (pH 5.5) was used directly. The result was that the membrane was not formed.

### [Comparative Example 2]

The same process as that in Example 1 was conducted except for one obtained by dropping the mixed basic buffer solution of sodium hydrogen carbonate and sodium carbonate in the 0.1 mass% sodium alginate aqueous solution to adjust the pH to 10 was used as the sodium alginate aqueous solution. The result was that the membrane was not formed.

### [Comparative Example 3]

The same process as that in Example 1 was conducted except for one obtained by dropping hydrochloric acid in the 0.1 mass% sodium alginate aqueous solution to adjust the pH to 1.5 was used as the sodium alginate aqueous solution. The result was that sodium alginate became insoluble and precipitated out, and the membrane was not formed.

**[Table 1]**

| | pH of anionic polymer aqueous solution | Thickness of membrane (nm) | Base |
|---|---|---|---|
| Example 1 | 3.0 | 80 | SiO₂ |
| Example 2 | 3.5 | 60 | SiO₂ |
| Example 3 | 4.0 | 40 | SiO₂ |
| Example 4 | 5.0 | 10 | SiO₂ |
| Example 5 | 3.0 | 85 | PET |
| Comparative Example 1 | 5.5 | 0 | SiO₂ |
| Comparative Example 2 | 10.0 | 0 | SiO₂ |
| Comparative Example 3 | 1.5 | 0 | SiO₂ |

When the pH of the solution containing the polyanion (anionic polymer) was in the range of 1.6 to 5.4, it was possible to produce the thin film with a base effectively by using the alternate immersion method (Figure 1, Table 1). It is believed to be because the pH in the range of 1.6 to 5.4 allowed the cationic group in the polycation (cationic polymer) to be positively charged effectively and promoted adsorption of the polyanion (anionic polymer).

Comparative Example 1 employed a polyanion (anionic polymer) solution in which the pH was not adjusted, which was one used in the conventional spin coating method. As is apparent from the result of Comparative Example 1, the alternate immersion method using such polyanion solution provided poor adsorption properties and the membrane was not formed. That is, the solution containing the polyanion and having a pH of 1.6 to 5.4 provides higher adsorption properties and the thin film produced by using such solution has properties different from those of the conventional thin film.

### [Example 6]

As the chitosan aqueous solution, the above described 0.1 mass% chitosan aqueous solution was used directly. As the sodium alginate aqueous solution, one obtained by adding 1 mass part of malic acid relative to 100 mass parts of the 0.1 mass% sodium alginate aqueous solution was used. The pH of the sodium alginate aqueous solution was 2.5.

A SiO₂ substrate (manufactured by ASAHI SANGYO KAISHA, Ltd., 5 inch silicon wafer: 30 mm x 70 mm x 1.0 mm thickness) was (i) immersed in the chitosan aqueous solution for 1 minute and then immersed in ultrapure water for rinse (specific resistance 18 MΩ·cm) for 1 minute, and (ii) immersed in the sodium alginate aqueous solution for 1 minute and then immersed in ultrapure water for rinse for 1 minute.

A procedure of repeating (i) and (ii) in order was taken as 1 cycle, and this cycle was repeated 30 times, thus obtaining a laminated membrane of chitosan and sodium alginate on the SiO₂ substrate. The thickness of the obtained laminated membrane was measured by filmetrics. The result was that the thickness of the membrane was 100 nm.

### [Example 7]

A laminated membrane was obtained by conducting the same process as that in Example 6 except for the cycle was repeated 23 times. The thickness of the obtained membrane was 75 nm.

### [Example 8]

The same process as that in Example 6 was conducted except for PET (manufactured by TOYOBO CO., LTD., A4100, thickness: 125 µm) was used as the base instead of the SiO₂ substrate. The thickness of the obtained membrane was 110 nm.

In the case of using PET as the base, it was possible to increase the thickness of the membrane in comparison with the case of using the SiO₂ substrate.

### [Example 9]

The same process as that in Example 6 was conducted except for one obtained by dropping 1 mass part of acetic acid relative to 100 mass parts of the 0.1 mass% sodium alginate aqueous solution was used as the sodium alginate aqueous solution. The pH of the sodium alginate aqueous solution was 3.5. The thickness of the obtained laminated membrane was 75 nm.

### [Comparative Example 4]

The same process as that in Example 6 was conducted except for one obtained by dropping 1 mass part of hydrochloric acid relative to 100 mass parts of the 0.1 mass% sodium alginate aqueous solution was used as the sodium alginate aqueous solution. The pH of the sodium alginate aqueous solution was 1.4. The result was that sodium alginate became insoluble and precipitated out, and the membrane was not formed.

### [Comparative Example 5]

The same process as that in Example 6 was conducted except for one obtained by dropping 1 mass part of nitric acid relative to 100 mass parts of the 0.1 mass% sodium alginate aqueous solution was used as the sodium alginate aqueous solution. The pH of the sodium alginate aqueous solution was 1.3. The result was that sodium alginate became insoluble and precipitated out, and the membrane was not formed.

**[Table 2]**

| | Acidic component | Number of cycle | Thickness of membrane (nm) | Base |
|---|---|---|---|---|
| Example 6 | Malic acid | 30 | 100 | SiO₂ |
| Example 7 | Malic acid | 23 | 75 | SiO₂ |
| Example 8 | Malic acid | 30 | 110 | PET |
| Example 9 | Acetic acid | 30 | 75 | SiO₂ |
| Comparative Example 4 | Hydrochloric acid | 30 | 0 | SiO₂ |
| Comparative Example 5 | Nitric acid | 30 | 0 | SiO₂ |

## Claims

1. A thin film with a base, comprising a base and a thin film formed on the base, wherein the thin film comprises a layer A formed by using a solution containing a polycation; and a layer B formed by using a solution containing a polyanion and having a pH of 1.6 to 5.4.

2. The thin film with a base according to claim 1, wherein the solution containing a polyanion and having a pH of 1.6 to 5.4 is a solution containing a polyanion and malic acid.

3. The thin film with a base according to claim 1 or 2, wherein the thin film is a thin film in which the layer A and the layer B are laminated alternately.

4. The thin film with a base according to any one of claims 1 to 3, wherein the polycation is a cationic polymer having two or more amino groups in one molecule.

5. The thin film with a base according to claim 4, wherein the cationic polymer is a basic polysaccharide or a derivative thereof, or a salt thereof.

6. The thin film with a base according to claim 5, wherein the basic polysaccharide is chitosan.

7. The thin film with a base according to any one of claims 1 to 6, wherein the polyanion is an anionic polymer having two or more carboxyl groups or carboxylate groups in one molecule.

8. The thin film with a base according to claim 7, wherein the anionic polymer is an acidic polysaccharide or a derivative thereof, or a salt thereof.

9. The thin film with a base according to claims 8, wherein the acidic polysaccharide is alginic acid.

10. A method for producing a thin film with a base, comprising:
a layer formation step of allowing a base to come into contact with a solution containing a polycation or a solution containing a polyanion and having a pH of 1.6 to 5.4 to form a layer derived from the polycation or the polyanion on the surface of the base; and
a laminating step of repeating
a step (i) of allowing a solution containing a polyanion and having a pH of 1.6 to 5.4 to come into contact with the layer derived from the polycation to form a layer derived from the polyanion on the layer derived from the polycation, and
a step (ii) of allowing a solution containing a polycation to come into contact with the layer derived from the polyanion to form a layer derived from the polycation on the layer derived from the polyanion.

11. The method according to claim 10, wherein the solution containing a polyanion and having a pH of 1.6 to 5.4 is a solution containing a polyanion and malic acid.

12. The method according to claim 10 or 11, wherein the laminating step is repeated until the numbers of the layer derived from the polycation and the layer derived from the polyanion are both 1 to 300.
